# EUROPÄISCHE PATENTSCHRIFT

(11) **EP 1 719 470 B1**
(45) Veröffentlichungstag und Bekanntmachung des Hinweises auf die Patenterteilung: **13.01.2016**
(21) Anmeldenummer: 06015678.3
(22) Anmeldetag: 05.06.2002
(51) Int. Cl.: A61B 18/00, A61F 9/008, A61B 18/20

(54) **Anordnung zum Materialabtrag**
System for ablation
Système pour l'ablation

(30) Priorität: 15.06.2001 DE 10129650
(43) Veröffentlichungstag der Anmeldung: 08.11.2006
(62) Teilanmeldung aus: 02732751.9
(73) Patentinhaber: Carl Zeiss Meditec AG, 07745 Jena (DE)
(72) Erfinder: Dick, Manfred, 07926 Gefell (DE); Elbrecht, Jens, 07751 Jena (DE); Schröder, Eckhard, 90542 Eckental (DE); Seitz, Bernhard, 07751 Jena-Wogau (DE)
(74) Vertreter: Niestroy, Manfred

(56) Entgegenhaltungen:
- WO-A-00/33912
- WO-A-98/57588
- DE-A1- 10 020 522
- US-A- 4 850 352

## Beschreibung

### Gebiet der Erfindung

Die Erfindung bezieht sich auf eine Anordnung zum Abtragen von Material mit Hilfe von Laserstrahlung.

### Stand der Technik

Verfahren und Anordnungen zum Abtragen von Material von der Oberfläche eines Gegenstandes mit Hilfe eines Laserstrahles, der auf die Oberfläche gerichtet ist, sind an sich bekannt. Darunter auch Verfahren und Anordnungen, bei denen der Laserstrahl scannend über die Oberfläche geführt und das Material dabei definiert abgetragen und dabei die Geometrie des Gegenstandes gezielt verändert wird.

Dabei kommt es insbesondere bei weichen, temperaturempfindlichen Materialien darauf an, die Laserenergie ohne wesentliche thermische Schädigung des Gebietes um den Abtragungsort einzubringen. Dies ist insbesondere dann von Bedeutung, wenn das abzutragende Material stark feuchtigkeitshaltig ist und eine Austrocknung des Materials infolge des Energieeintrages verhindert werden soll, um zu vermeiden, daß sich die Materialeigenschaften oder auch die Bedingungen für den Materialabtrag, wie etwa die Abtragungsgeschwindigkeit, während des Abtragungsprozesses in unerwünschter Weise verändern.

Das ist beispielsweise der Fall, wenn die Oberflächenkrümmung einer künstlichen Kontaktlinse verstärkt oder verringert werden soll mit dem Ziel, die Sehkraft eines menschlichen Auges mit dieser Kontaktlinse zu korrigieren. Aber auch bei der Bearbeitung von totem oder lebendem biologischem Gewebe wie Knorpel, Zahnhartgewebe oder auch in der Augenchirurgie bei der Formung der Hornhaut (photorefraktive Keratektomie) kommt es nicht nur auf Sorgfalt bei der Formgebung, sondern auch darauf an, die Eigenschaften des verbleibenden Materials beizubehalten.

Von besonderer Bedeutung für einen definierten Materialabtrag bei den vorgenannten Anwendungen ist die Konstanthaltung der klimatischen Bedingungen in der Umgebung des Abtragungsortes über die Dauer des Materialabtrages hinweg, die vor allem bestimmt sind von der Temperatur und der Feuchtigkeit an der Materialoberfläche bzw. in deren unmittelbarer Nähe.

Von weiterer Bedeutung für den definierten Materialabtrag ist aber auch die Kontinuität des Energieeintrages in das Material. Da die Laserstrahlung auf dem Weg zwischen einer Abstrahlungsoptik und dem Abtragungsort die freie Atmosphäre oder gegebenenfalls auch ein Schutzgas durchquert, ist es möglich, daß die bei der Abtragung des Materials entstehenden Abprodukte, wie Rauch oder Materialpartikel, die Atmosphäre in unmittelbarer Nähe des Abtragungsortes beeinträchtigen und dabei auch, indem sie den Laserstrahl durchqueren, die Intensität der Laserstrahlung in undefinierbarer Weise schwächen.

Aus US 5,344, 418 ist eine Anordnung bekannt, bei der nahe der Austrittsöffnung für den Laserstrahl aus einem Gerät zur Materialabtragung Strömungskanäle vorgesehen sind, aus denen während des Materialabtrages ein Gas- bzw. Luftstrom auf den Abtragungsort gerichtet ist, womit erreicht wird, daß Rauch und Materialpartikel vom Abtragungsort weggeblasen werden. Nachteilig dabei ist allerdings, daß der Gas- bzw. Luftstrom die Materialoberfläche am Abtragungsort überstreicht, was zur Folge hat, daß sowohl ein auf der Oberfläche vorhandener Feuchtigkeitsfilm zerstört und damit dessen Schutzfunktion aufgehoben wird als auch insbesondere innerhalb von feuchtehaltigem, stark hygroskopischem Material eine Austrocknung in unzulässigem Maße erfolgt bzw. die Hydratationszustände im Material während des Abtrages einer unerwünschten Beeinflussung unterworfen sind.

Bei einer in US 5,181,916 beschriebenen Einrichtung werden die Verunreinigungen wie Rauch oder Materialpartikel nicht weggeblasen, sondern mit Hilfe eines Gasstromes abgesaugt. Zu diesem Zweck ist eine Ansaugöffnung konzentrisch um eine Mündungsöffnung eines Gerätes angeordnet, aus dem der Laserstrahl austritt und auf den Abtragungsort gerichtet ist. Doch auch hier hat das über den Behandlungsort strömende Gas zur Folge, daß sowohl die Feuchte an der Materialoberfläche abtransportiert wird als auch das Material zumindest nahe dem Abtragungsort austrocknet.

In DE 100 20 522 A1 ist eine Anordnung zur Absaugung von Abprodukten bei der Ablation von biologischem Gewebe beschrieben. Hier ist der Laserstrahl durch einen röhrenförmigen Kanal hindurch auf das Gewebe gerichtet und die Abprodukte werden in den Kanal hinein abgesaugt. Es wird innerhalb des Kanals ein gegenläufig zur Laserstrahlung gerichteter Luftstrom erzeugt, wobei die abgesaugte Luft nicht aus der Umgebung des Abtragungsortes kommt, sondern aus Zuführöffnungen strömt, die nahe der Mündungsöffnung im Kanal vorhanden sind. Das hat zur Folge, daß die Materialoberfläche nicht vom Luftstrom überstrichen und so die Austrocknung vermieden wird. Außerdem ist der Luftstrom vom Zentrum des Kanals, in dem der Laserstrahl verläuft, radial nach außen gerichtet, so daß Rauch und Materialpartikel vom Zentrum und damit vom Laserstrahl ferngehalten werden und somit verhindert wird, daß die Intensität der Laserstrahlung durch derartige Verunreinigungen in unerwünschter Weise beeinflußt wird.

Allerdings gelingt es auch hiermit noch nicht, die Materialabtragung mit Laserenergie, insbesondere im Hinblick auf die Feinbearbeitung von Oberflächen, von allen Umgebungseinflüssen freizuhalten. Die Abtragungsbedingungen werden trotz der vorstehend genannten Maßnahmen immer noch durch sich während des Abtragungsvorganges verändernde Temperatur und Luftfeuchte am Abtragungsort beeinflußt. Um eine höhere Genauigkeit bei der Formgebung durch Materialabtrag erzielen zu können, besteht nach wie vor das Bedürfnis, derartige Einflüsse zu reduzieren.

### Beschreibung der Erfindung

Davon ausgehend liegt der Erfindung die Aufgabe zugrunde, unter Beibehaltung oder auch Verbesserung der bereits bekannten Maßnahmen zur Freihaltung des Laserstrahlquerschnitts von Verunreinigungen auch die klimatischen Umgebungsbedingungen am Abtragungsort über die gesamte Zeit des Abtragungsprozesses hinweg konstant zu halten.

Die Erfindung wird durch den unabhängigen Anspruch 1 definiert, vorteilhafte Ausführungen werden in den Unteransprüchen beschrieben.

Bei einem Verfahren der eingangs genannten Art wird die Temperatur und/oder die Feuchtigkeit am Abtragungsort und/oder in dessen unmittelbarer Nähe mittels eines während der Dauer des Abtragungsprozesses in vorgegebener Richtung über den Abtragungsort hinweg strömenden Gases im wesentlichen konstant gehalten. Das Gas hat dabei eine vorgegebene Temperatur, einen vorgegebenen Feuchtigkeitsgehalt und/oder eine vorgegebene Strömungsgeschwindigkeit.

Bei einer ersten Ausgestaltung der Erfindung wird während der gesamten Dauer des Abtragungsprozesses ein Luftstrom mit konstanter Temperatur, konstantem Feuchtigkeitsgehalt und konstanter Strömungsgeschwindigkeit über den Abtragungsort hinweg geführt.

Damit wird unter Nutzung der Luft als Transportmedium bei entsprechend vorgewählter, unter der Solltemperatur am Abtragungsort liegender Temperatur der auf den Abtragungsort gerichteten Luft überschüssige Wärmeenergie abtransportiert. Umgekehrt hat die auf den Abtragungsort gerichtete Luft eine verhältnismäßig hohe relative Feuchte, so daß die Zufuhr von Feuchtigkeit gewährleistet ist und der Austrocknung an der Materialoberfläche und innerhalb des Materials entgegengewirkt wird. Beispielsweise kann der Luftstrom mit einer Temperatur von 37° und einer relativen Luftfeuchte von 100% bei einer Strömungsgeschwindigkeit von etwa 0,5m/s zum Abtragungsort gerichtet werden.

In Abhängigkeit von den Eigenschaften des zu bearbeitenden Materials kann es sich als günstig erweisen, wenn der Luftstrom mit einer Temperatur im Bereich von - 20°C bis 30°C, einer relativen Luftfeuchte im Bereich von 0-100% und einer Strömungsgeschwindigkeit im Bereich von 1 m/s bis 10 m/s über den Abtragungsort hinweggeführt wird. Eine häufig bevorzugte Variante besteht darin, Luft einer Temperatur von -8°C und einer relativen Feuchte von 80 % mit ca. 3m/s über den Abtragungsort hinwegzuführen.

Damit gelingt es, die klimatischen Bedingungen während der Abtragung in verhältnismäßig engen Grenzen konstant zu halten. Wird beispielsweise während des Abtragungsprozesses eine Leistung von ca. 0,5 Watt scannend kontinuierlich in das Material eingetragen, wird zwar der überwiegende Teil dieser Leistung für die Ablation verbraucht, jedoch wird ein nicht unerheblicher Leistungsanteil in thermische Energie umgewandelt, die aber nach dem erfindungsgemäßen Verfahren weitestgehend abtransportiert wird, so daß wie bereits beschrieben das stationäre Gleichgewicht im wesentlichen erhalten bleibt.

Im Rahmen der Offenbarung liegt es weiterhin, wenn die Strömungsgeschwindigkeit und die Fördermenge des Luftstrom in Abhängigkeit von der Impulsfolgefrequenz der zur Ablation genutzten Laserstrahlung so vorgegeben wird, daß das während einer Impulsfolge ablatierte Gewebe innerhalb des Zeitraumes, der bis zum Beginn der nachfolgenden Impulsfolge vergeht, mit dem Luftstrom abtransportiert werden kann. Dies ist bei einer Impulsfolgefrequenz von 1 kHz und einer am Abtragungsort bearbeiteten Flächengröße von beispielsweise 8 mm² mit einer Strömungsgeschwindigkeit von ca. 8m/s möglich, wobei die Luftmenge etwa 40 cm³/s betragen sollte. Hierbei kann ein Schlauch mit etwa 8 mm Durchmesser genutzt werden.

In einer bevorzugten Ausgestaltung der Erfindung ist vorgesehen, daß ein Luftstrom mit konstanter Temperatur und konstantem Feuchtigkeitsgehalt, jedoch mit während der Dauer des Abtragungsprozesses zunehmender Strömungsgeschwindigkeit über den Abtragungsort hinweg geführt wird. Auch hierbei kann der Luftstrom beispielsweise eine Temperatur von 37° und eine relative Luftfeuchte von etwa 100% haben, wobei jedoch zu Beginn der Abtragung die Strömungsgeschwindigkeit ca. 0,2m/s beträgt und über die Dauer der Abtragung hinweg bis zu 10m/s erhöht wird. Auf diese Weise gelingt es, auch bei Energieeinträgen mit höheren Leistungswerten die überschüssige thermische Energie abzuführen.

Im Rahmen der Erfindung liegt es auch, den Luftstrom mit konstanter Strömungsgeschwindigkeit über den Abtragungsort hinweg zuführen, dagegen aber während des Abtragungsprozesses die Temperatur der strömenden Luft zu verringern und/oder ihre relative Feuchte zu erhöhen. Diesbezüglich kann beispielsweise vorgesehen sein, daß die Strömungsgeschwindigkeit der Luft über den gesamten Abtragungsprozeß hinweg 0,5m/s beträgt, während die Lufttemperatur innerhalb eines Bereiches von etwa 42°C zu Anfang bis zu etwa 10°C bei Ende des Abtragungsprozesses verändert wird und die Luftfeuchte von etwa 80% zu Anfang bis auf 100% bei Ende des Abtragungsprozesses verändert wird. Damit werden noch bessere Ergebnisse bei der Einstellung eines Temperatur- und Feuchtigkeitsgleichgewichtes zwischen Material und der klimatisierten Umgebung an der Materialoberfläche erzielt als bei konstanten Temperatur- und Feuchtewerten, wodurch sich weiterhin verbesserte reproduzierbare Bedingungen bei Formgebung ergeben.

In diesbezüglichen Ausgestaltungsvarianten ist es möglich, die Veränderungen von Temperatur und/oder Luftfeuchte während des Abtragungsprozesses sowohl kontinuierlich als auch anhand vorgegebener, diskontinuierlicher Zeitplan-Funktionen vorzunehmen.

Alternativ hierzu ist es auch denkbar, die Veränderung der Temperatur, der relativen Feuchte und/oder der Strömungsgeschwindigkeit der Luft in Abhängigkeit von Temperatur- und/oder Feuchtigkeitswerten vorzunehmen, die während des Abtragungsprozesses unmittelbar am oder nahe dem Abtragungsort wiederholt gemessen, ausgewertet und als Führungsgrößen für vorzunehmende Veränderungen des Luftstrom genutzt werden. So liefert beispielsweise eine kontinuierliche Messung von Temperatur und Feuchtigkeit am Abtragungsort Informationen, die dazu genutzt werden können, die Temperatur der Luft zu verringern bzw. deren Feuchte zu erhöhen oder auch die Strömungsgeschwindigkeit zu verändern, um in geeigneter Weise stetig aktiv auf die Konstanthaltung der klimatischen Bedingungen am Abtragungsort Einfluß nehmen zu können.

In Verbindung mit den vorgenannten Maßnahmen zur Konstanthaltung der Umgebungsbedingungen ist außerdem der Luftstrom stets so gerichtet, daß die während der Abtragung entstehenden Abprodukte, wie Rauch und Materialpartikel, vom Luftstrom erfaßt und mit der strömenden Luft vom Abtragungsort entfernt werden, ohne dabei den auf den Abtragungsort gerichteten Laserstrahl zu durchqueren.

Es sei ausdrücklich darauf hingewiesen, daß sich die Erfindung nicht auf die Verwendung von Luft als Transportmittel von Wärmeenergie und Feuchtigkeit beschränkt, sondern daß darüber hinaus auch jedes andere geeignete Gas, wie beispielsweise Stickstoff, verwendet werden kann.

Das offenbarte Verfahren ist bevorzugt zur Veränderung der Oberflächenkrümmung von künstlichen Kontaktlinsen geeignet mit dem Ziel, je nach Verstärkung oder Verringerung der Krümmung die Fehlsichtigkeit eines menschlichen Auges zu korrigieren. Dabei besteht ein wesentlicher Vorteil darin, daß die Bearbeitung bei Abwesenheit des späteren Kontaktlinsenträgers erfolgen kann.

Offenbart sind weiterhin Anordnungen zum Abtragen von Material, die zur Ausübung der vorgenannten Verfahrensschritte geeignet sind und in der beschriebenen Weise die Bearbeitung sowohl von künstlichen als auch natürlichen Materialien, darunter auch biologischem Gewebe, erlauben. Bei diesen Anordnungen sind Mittel vorgesehen, mit denen während der Einwirkung der Laserenergie ein Gasstrom über den Abtragungsort hinweg geführt wird, wobei dessen Temperatur, relative Feuchte und/oder Strömungsgeschwindigkeit des über den Abtragungsort hinwegströmenden Gases vorgegeben werden können. Als Gas kommt hier bevorzugt Luft zur Anwendung, jedoch sind auch andere Gase geeignet, wie beispielsweise Stickstoff.

In einer besonders bevorzugten Ausgestaltung sind die Anordnungen ausgestattet mit Mitteln zu Vorauswahl der Temperatur, der relativen Luftfeuchte und/oder der Strömungsgeschwindigkeit aus vorgegebenen Wertebereichen. Die Auswahl kann vor Beginn des Abtragungsprozesses stattfinden, wobei Einrichtungen zum Konstanthalten der vorausgewählten Werte während des gesamten Abtragungsprozesses vorhanden sind.

Weiterhin ist vorgesehen, daß die Mittel bzw. Einrichtungen zur Vorauswahl oder Veränderung der Temperatur, der relativen Luftfeuchte und/oder der Strömungsgeschwindigkeit mit einer Ansteuerschaltung gekoppelt sind, die beispielsweise in Abhängigkeit von Werten, die nach einer Zeitplan-Funktion vorgegeben werden, Stellsignale abgeben. Diese Ansteuerschaltung kann weiterhin mit einer Lufterwärmungseinrichtung und/oder mit einer Luftbefeuchtungseinrichtung gekoppelt sein.

Die Luftbefeuchtungseinrichtung sollte vorteilhaft mit einem Vernebler, bevorzugt mit einem Ultraschallvernebler ausgestattet sein, der Feuchtigkeit mit einer konstanten Tröpfchengröße von < 4 µm abgibt. Dies gilt beispielsweise für die refraktive Laserchirurgie unter Nutzung einer Laserstrahlung der Wellenlänge von 193 nm, wobei die Vernebelungsleistung 0,5 bis 2 ml/min betragen sollte. Damit wird eine im Hinblick auf die benötigte Anfeuchtung und geringste Wasserausscheidung am Operationsort optimierte Nebeldichte erreicht.

Weiterhin sind Mittel vorgesehen, die die Strömungsrichtung des Gases bzw. der Luft derart beeinflussen, daß der Laserstrahlquerschnitt nicht durch Ablationsabprodukte durchquert und dadurch die Strahlungsintensität in undefinierbarer Weise beeinflußt wird. Diesbezüglich sind beispielhaft zwei jeweils ringförmig zentrisch um den Laserstrahl angeordnete Strömungskanäle in Richtung der Laserstrahlung aufeinander folgende vorhanden, von denen einer mit Austrittsöffnungen, der andere mit Eintrittsöffnungen für den Luftstrom ausgestattet sind. Dabei sind die Austrittsöffnungen eines ersten der beiden Strömungskanäle und die Eintrittsöffnungen des anderen Strömungskanals so positioniert, daß der Luftstrom im wesentlichen parallel zur Laserstrahlung gerichtet ist, bevorzugt mit einer Strömungsrichtung entgegengesetzt zur Richtung der Laserstrahlung.

Je nach Anwendungsfall kann es sich weiterhin als vorteilhaft erweisen, wenn die Richtung der zuströmenden Luft mit der Tangente über dem Abtragungsort einen Winkel von 0-70° einschließt und die Eintrittsöffnungen für die Absaugung des Luftstromes vom Abtragungsort so ausgebildet sind, daß die Richtung der abströmenden Luft mit der Tangente über dem Abtragungsort ebenfalls einen Winkel im Bereich zwischen 0° und 70° einschließt.

Zur Ermittlung der Feuchtigkeitswerte am Abtragungsort ist beispielsweise eine Streulichtmeßeinrichtung vorgesehen, bei der die Intensität der Reflexion eines gesonderten, auf die Materialoberfläche gerichteten Laserstrahls, dessen Wellenlänge im sichtbaren oder infraroten Spektralbereich liegt, als Maß der Feuchtigkeit an der Oberfläche genutzt wird. Die physikalischen Parameter dieser gesonderten Laserstrahlung, insbesondere Intensität und Wellenlänge, sind dabei in Abhängigkeit von den Eigenschaften des zu bearbeitenden Materials so gewählt, daß keine Veränderung der Materialeigenschaften aufgrund dieser Strahlung eintritt. Zum kontaktfreien Messen der aktuellen Temperaturen an der Materialoberfläche während des Abtragungsprozesses kann eine handelsübliche Thermokamera vorgesehen sein.

### Kurze Beschreibung der Zeichnungen

Die Erfindung soll nachfolgend anhand eines Ausführungsbeispieles näher erläutert werden. In den zugehörigen Zeichnungen zeigen
- Fig.1: eine nicht erfindungsgemäsze Anordnung zum Materialabtrag mit Hilfe von Laserstrahlung, bei der ein Laserstrahl auf die Materialoberfläche gerichtet und eine Einrichtung zur Klimatisierung der Umgebung an der Oberfläche zur Zuführung eines klimatisierten Luftstromes vorgesehen ist,
- Fig.2: ein Beispiel für die Ausgestaltung einer Zu- und Abführeinrichtung für einen klimatisierten Luftstrom zur Materialoberfläche und deren Anordnung in der Nähe des Abtragungsortes,
- Fig.3: eine Möglichkeit für die Positionierung von Meßeinrichtungen zur Ermittlung von Temperatur- und Feuchtigkeitswerten aus der Nähe des Abtragungsortes.
- Fig.4: eine weitere Ausgestaltungsmöglichkeit der Einrichtung zur Zuführung und Abführung eines klimatisierten Luftstromes.

### Ausführliche Beschreibung der Zeichnungen

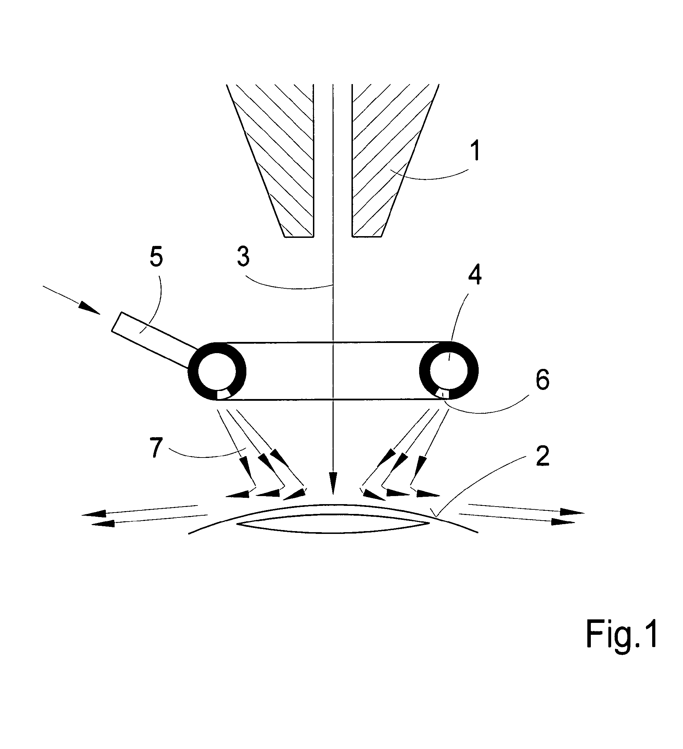

In Fig.1 ist ein röhrenförmiger Kanal 1 dargestellt, aus dessen Ende ein Laserstrahl 3 austritt und auf die Oberfläche eines Gegenstandes 2 gerichtet ist. Eine solche Anordnung kann beispielsweise zur Veränderung der Krümmung von Kontaktlinsen oder auch zur photorefraktiven Keratektomie genutzt werden, bei der die Krümmung der Hornhaut eines menschlichen Auges unter Einwirkung der Laserstrahlung gezielt durch Abtrag des biologischen Gewebes der Hornhaut korrigiert wird.

Es sei angenommen, daß während der Dauer des Ablationsprozesses ein Leistungseintrag in das Gewebe von ca. 0,5 Watt erfolgt. Dabei wird ein Großteil der Leistung für die Ablation verbraucht, ein nicht unerheblicher Teil jedoch wird in unerwünschte thermische, akustische und Fluoreszenzenergie umgewandelt. Dabei würden vor allem die thermischen Verlustanteile die Abtragungsbedingungen stören, da hierdurch der Tränenfilm, also die Feuchtigkeit auf der Hornhaut sowie die Hydratationseigenschaften der Hornhaut selbst beeinflußt werden.

Um eine definierte Abtragungsrate und damit die Möglichkeit einer definierten Formgebung der Hornhautoberfläche erzielen zu können, soll der Aufgabe der Erfindung entsprechend der unerwünschte Einfluß auf die Ablationsbedingungen verringert bzw. nach Möglichkeit ganz aufgehoben werden, indem die klimatischen Umgebungsbedingungen konstant gehalten werden.

Erfindungsgemäß ist diesbezüglich ein röhrenförmiger Kanal 4 vorgesehen, der über einen Anschlußstutzen 5 und eine hieran angeschlossene (nicht dargestellte) Verbindungsleitung mit einer Luftfördereinrichtung (ebenfalls nicht dargestellt) in Verbindung steht.

Die Luftfördereinrichtung sorgt dafür, daß dem röhrenförmigen Strömungskanal 4 Luft zugeführt wird, die durch Austrittsöffnungen 6 aus dem Strömungskanal 4 austritt.

Dabei sind die Austrittsöffnungen 6 so angeordnet, daß die Strömungsrichtungen 7 der austretenden Luft, mit dem Laserstrahl 3 einem spitzen Winkel einschließend, zur Oberfläche des Gegenstandes 2 hin gerichtet sind.

Der Strömungskanal 4 ist ringförmig gekrümmt und zentrisch um den Laserstrahl 3 angeordnet, während die Austrittsöffnungen 6 radialsymmetrisch um den Laserstrahl verteilt positioniert sind, so daß die Strömungsrichtungen 7 in ihrer Gesamtheit etwa einen Kreiskegelmantel bilden, in dessen Zentrum der Laserstrahl 3 verläuft.

Der Abstand des Strömungskanals 4 zum Gegenstand 2 ist so bemessen, daß die Spitze dieses Kreiskegelmantels etwa mit dem Auftreffort des Laserstrahles 3 auf dem Gegenstand 2 zusammenfällt.

Damit wird erreicht, daß Strömungsrichtungen 7 etwa am Auftreffort des Laserstrahles 3 auf der Gegenstand 2 aufeinandertreffen und sich dabei gegenseitig so beeinflussen, daß sich die Strömungsrichtungen 7 umkehren und die strömende Luft radial nach außen abgeführt wird. Das hat zur Folge, daß Ablationsabprodukte wie Rauch und ablatierte Gewebepartikel vom Luftstrom erfaßt und mit der Luft in radialer Richtung abtransportiert werden.

Damit wird weitestgehend erreicht, daß die Ablationsprodukte den Laserstrahl 3 nicht durchqueren und so auch die Laserstrahlungsintensität nicht beeinträchtigen können.

Außerdem ist erfindungsgemäß vorgesehen, daß die Luftfördereinrichtung mit einer Klimatisierungseinrichtung für die zum Strömungskanal 4 geleitete Luft gekoppelt ist. Die Klimatisierungseinrichtung ist so ausgebildet, daß Temperatur und relative Feuchte der Luft beeinflußt werden können. Außerdem sind Mittel vorhanden, mit denen die Temperaturwerte, Werte für die relative Luftfeuchte sowie auch Werte für die je Zeiteinheit geförderte Luftmenge vor Beginn des Ablationsprozesses voreingestellt werden können. Diesbezüglich ist sowohl die Luftfördereinrichtung als auch die Klimatisierungseinrichtung mit Mitteln zur Befehlseingabe, beispielsweise Tastern, Schaltern oder Drehgebern ausgestattet, die Teil einer Ansteuerung sind. Derartige Einrichtungen zur Luftförderung und zur Klimatisierung der Luft sowie entsprechende Eingabemittel sind aus dem Stand der Technik bekannt und müssen deshalb hier nicht näher erläutert werden.

Ist beispielsweise als Quelle für die Laserstrahlung ein Excimer-Laser, vorzugsweise des Typs MEL 70 G-Scan, vorgesehen, der die sehr sensible Laserstrahlung mit einer Wellenlänge von 193 nm abgibt, so kann erfindungsgemäß durch Vorauswahl einer Temperatur von 37°C, einer relativen Luftfeuchte von etwa 100% und einer Strömungsgeschwindigkeit von etwa 0,5m/s dafür gesorgt werden, daß die klimatischen Umgebungsbedingungen um den Abtragungsort während des Ablationsprozesses in verhältnismäßig engen Grenzen konstant bleiben. Damit wird auch eine verhältnismäßig konstante Abtragungsrate gewährleistet, womit die erforderliche Präzision bei der Formgebung weitestgehend erreicht ist.

Zusätzlich können sowohl die Luftfördereinrichtungen als auch die Klimatisierungseinrichtungen noch mit Mitteln zur Konstanthaltung der vorgewählten Werte ausgestattet sein. Derartige Einrichtungen, die sowohl die Temperatur, die Luftfeuchte als auch die Strömungsgeschwindigkeit von Luft konstant halten, sind ebenfalls aus dem Stand der Technik bekannt und werden deshalb hier nicht näher erläutert.

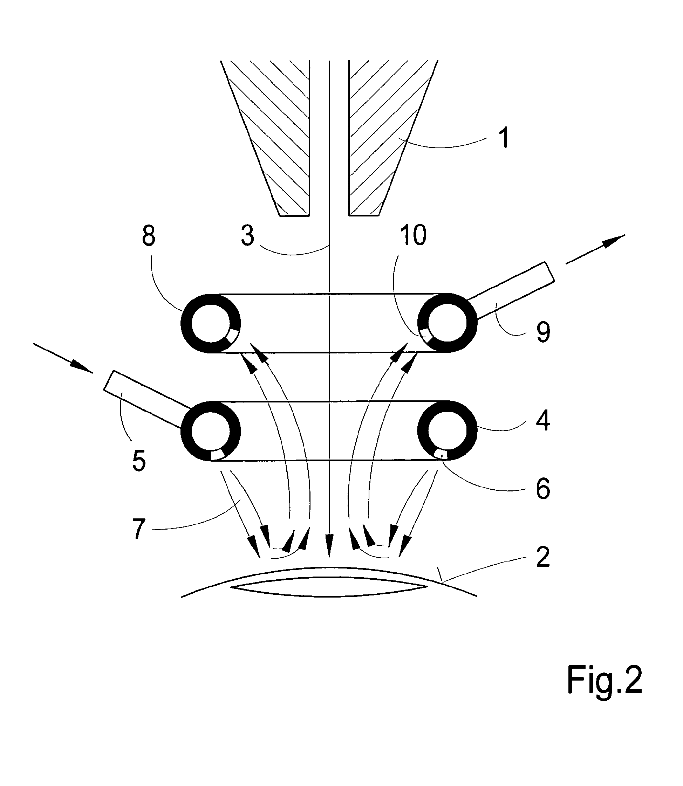

In einem in Fig.2 dargestellten Ausgestaltungsbeispiel der erfindungsgemäßen Anordnung ist neben dem röhrenförmigen Strömungskanal 4 ein weiterer röhrenförmiger Strömungskanal 8 vorgesehen, der ebenso wie der Strömungskanal 4 ringförmig um den Laserstrahl 3 angeordnet ist, der sich jedoch in einer größeren Entfernung als der Strömungskanal 4 von dem Gegenstand 2 befindet und im Gegensatz zum Strömungskanal 4 nicht mit einer Luftfördereinrichtung zur Zuführung von Luft, sondern mit einer Absaugeinrichtung (zeichnerisch nicht dargestellt) verbunden ist, die über eine Schlauchleitung (ebenfalls zeichnerisch nicht dargestellt) und einen Anschlußstutzen 9 mit dem Strömungskanal 8 in Verbindung steht.

Der Strömungskanal 8 verfügt über Eintrittsöffnungen 10, die im wesentlichen in gleicher Ordnung wie die Austrittsöffnungen 6 am Strömungskanal 4 positioniert sind.

Beim Betreiben dieser Anordnung entsteht rings um den Laserstrahl 3 eine Luftströmung, die zunächst aus den Austrittsöffnungen 6 des Strömungskanals 4 zum Gegenstand 2 und danach vom Gegenstand 2 zu den Einströmöffnungen 10 des Strömungskanals 8 hingerichtet ist.

Im Gegensatz zur Ausgestaltungsvariante nach Fig.1 wird mit dieser Anordnung dafür gesorgt, daß die Ablationsabprodukte nicht radial vom Laserstrahl 3 nach außen abtransportiert werden, sondern (etwa in entgegengesetzter Richtung zum Laserstrahl 3) durch die Eintrittsöffnungen 10 hindurch in den Strömungskanal 8 hinein und von dort zur Absaugeinrichtung abtransportiert werden. Dies hat zur Folge, daß die Ablationsabprodukte (Rauch, Gewebepartikel) den Laserstrahl 3 nicht durchqueren können und außerdem auch nicht die Umgebung des Ablationsortes verunreinigen bzw. zu Geruchsbelästigungen für die bearbeitende Person führen.

Hierbei ist vorgesehen, daß die Luft mit vorgegebener, konstant gehaltener Temperatur, relativen Feuchte und Strömungsgeschwindigkeit aus den Ausströmöffnungen 6 austritt und auf diese Weise für definierte klimatische Bedingungen an dem Gegenstand 2 sorgt.

Dagegen kann in einer weiteren Ausgestaltung der Anordnung vorgesehen sein, daß die Temperatur- und Feuchtigkeitswerte der Luft sowie deren Strömungsgeschwindigkeit während des Abtragungsprozesses nicht konstant gehalten werden, sondern daß Meßwertaufnehmer zur Erfassung von Temperatur- und Feuchtigkeitswerten aus unmittelbarer Nähe des Abtragungsortes vorhanden und über eine Ansteuerung mit der Luftfördereinrichtung und der Klimatisierungseinrichtung in Verbindung stehen.

Damit ist es möglich, auf aktuelle Veränderungen der klimatischen Umgebungsbedingungen sehr schnell zu reagieren, indem auf Grundlage der ermittelten Werte die Erhöhung oder Verringerung der Temperatur der strömenden Luft oder auch deren relativer Feuchte veranlaßt wird, um damit in noch engeren Grenzen der Auswirkung der thermischen Verlustleistung entgegenwirken zu können.

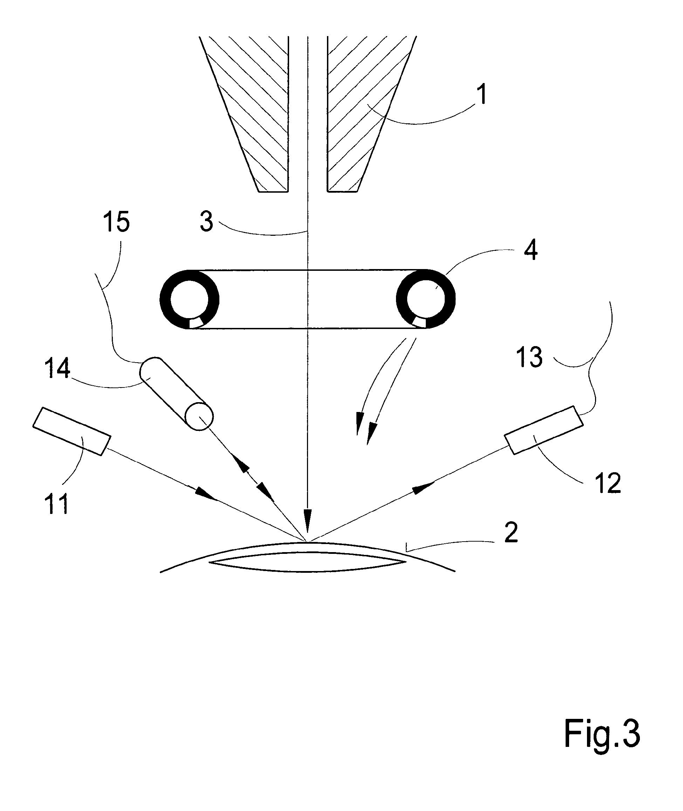

Beispiele für die Anordnung solcher Meßwertgeber sind in Fig.3 dargestellt. Hier ist beispielsweise zum Messen der Feuchtigkeitswerte am Abtragungsort eine Streulichtmeßeinrichtung vorgesehen, bestehend aus einer Laserdiode 11, die Licht im sichtbaren oder infraroten Spektralbereich auf den Gegenstand 2 richtet, und aus einem Fotodetektor 12, der die Reflexion des von der Laserdiode 11 ausgesendeten Laserstrahlung empfängt und dessen Ausgangssignale ein Maß für die Feuchtigkeit an der Hornhautoberfläche sind.

Über einen Signalweg 1 3 steht die Fotodiode 12 über eine Auswerte- und Ansteuerschaltung (nicht dargestellt) mit der Klimatisierungseinrichtung in Verbindung. Die reflektierte gestreute Intensität der von der Fotodiode 11 ausgehenden Laserstrahlung wird wesentlich davon bestimmt, ob sich noch ein Feuchtigkeitsfilm auf der Hornhaut-oberfläche befindet bzw. wie weit bereits eine Austrocknung erfolgt ist.

Zur Gewinnung von Temperaturwerten aus der unmittelbaren Nähe des Abtragungsortes kann eine handelsübliche Temperaturfernmeßeinrichtung, etwa eine Thermokamera, verwendet und mit ihrer Meßrichtung so ausgerichtet werden, daß damit die Temperaturwerte vom Abtragungsort erfaßt und über einen Signalweg 14 an die Auswerte- und Ansteuerschaltung weitergegeben werden, die mit der Klimatisierungseinrichtung in Verbindung steht.

Mit der vorliegenden Erfindung wird erreicht, daß mit einer kompakten Anordnung in der unmittelbaren Nähe des Behandlungsortes, beispielsweise des zu behandelnden Auges bei der photorefraktiven Keratektomie, neben der Absaugung der Ablationsabprodukte eine definierte Temperatur und Luftfeuchte eingestellt werden kann und damit bewirkt wird, daß die Abtragungseigenschaften des Hornhautgewebes konstant gehalten werden. Wie ausführlich dargelegt, erfolgt die Einstellung des stationären Gleichgewichtes von Luftfeuchte und Temperatur während der Laserbehandlung durch gezielte Zu- und Abfuhr temperierter, befeuchteter Luft bei definierter Strömungsgeschwindigkeit in der unmittelbaren Nähe des Behandlungsortes.

Mit einer bevorzugten Ausgestaltung wird mit gesättigter Luft, die etwa auf Körpertemperatur erwärmt ist, und einer verhältnismäßig geringen Strömungsgeschwindigkeit von etwa 0,5 m/s über den gesamten Verlauf des Ablationsprozesses hinweg gearbeitet. Ein zu Beginn des Abtragungsprozesses noch auf den Gegenstand 2 vorhandener Feuchtigkeitsfilm bedingt eine zunächst geringe Ablationsrate. Da aber während des Ablationsprozesses thermische Energie frei wird, führt diese dazu, daß der Feuchtigkeitsfilm zunehmend austrocknet und sich die Ablationsrate dadurch erhöht. Dem wird in der beschriebenen Art und Weise entgegengewirkt.

Bei der Arbeit mit einem Excimer-Laser als Strahlungsquelle für die Wellenlänge von 193 nm ist zu beachten, daß die Absorption für diese Wellenlänge in Wasser deutlich höher ist als in Luft bzw. einem anderen Spülgas, wie beispielsweise etwa Stickstoff. Daraus wird deutlich, daß es erforderlich ist, der Austrocknung entgegenzuwirken, um zu konstanten Abtragungsbedingungen zu kommen. Insofern gelingt es mit der erfindungsgemäßen Anordnung, stets ein Temperatur- und Feuchtigkeitsgleichgewicht zwischen dem Gegenstand (Kontaktlinse oder Hornhaut) und klimatisierter Umgebung an dessen Oberfläche einzustellen. Störgrößen wie ein anfänglich stärker vorhandener Feuchtigkeitsfilm sowie die mit dem Energieeintrag zunehmende Austrocknung werden mit den erfindungsgemäß vorgeschlagenen Mitteln ausgeglichen.

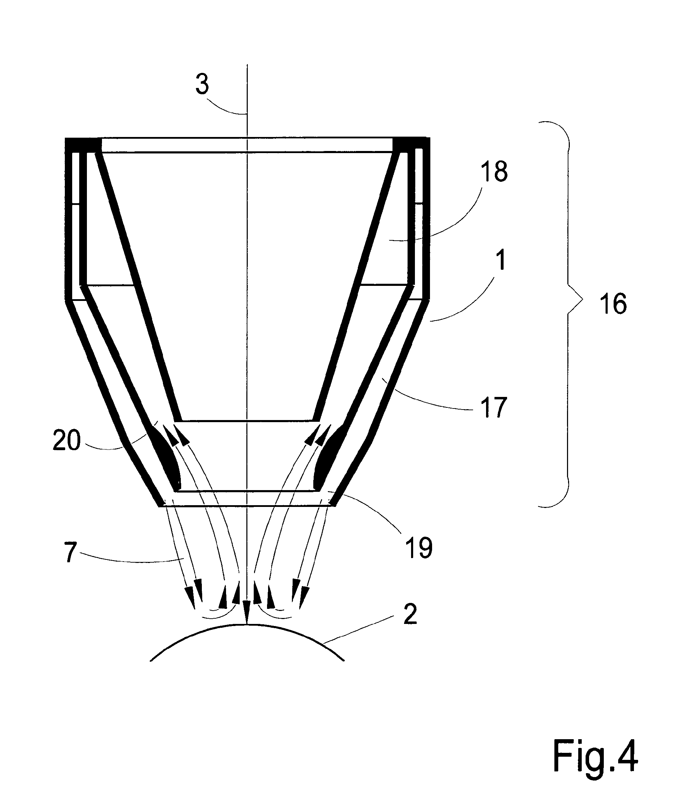

Fig.4 zeigt eine weitere Ausgestaltungsmöglichkeit hinsichtlich der Zuführung und Abführung eines klimatisierten Luftstromes 7 zur bzw. von der Oberfläche des Gegenstandes 2. Wie aus Fig.4 hervorgeht, weist das dem Gegenstand 2 zugewandte Ende des röhrenförmigen Kanals 1 einen konisch verlaufenden Abschnitt 16 mit zwei den Laserstrahl 3 konzentrisch umschließenden Kammern 17 und 18 auf. Die Kammer 17, die in eine ringförmige Ausströmöffnung 19 mündet, steht mit einer Luftklimatisierungs- und -fördereinrichtung (zeichnerisch nicht dargestellt) in Verbindung, die der Erzeugung eines Überdrucks aus klimatisierter Luft in der Kammer 17 dient. Die ringförmige Ausströmöffnung 19 ist so gestaltet, daß der aufgrund des Überdrucks austretende klimatisierte Luftstrom 7 auf die Oberfläche des Gegenstandes 2 gerichtet ist, wo er reflektiert wird.

Die Kammer 18 ist mit einer Absaugeinrichtung (zeichnerisch nicht dargestellt) verbunden, die einen Unterdruck erzeugt, und sie weist eine ringförmige Einströmöffnung 20 auf, durch welche der von der Oberfläche des Gegenstandes 2 reflektierte Luftstrom 7 in die Kammer 18 hineingesaugt und zur Absaugeinrichtung abtransportiert wird.

Zur Verbindung der Kammer 17 mit der Luftklimatisierungs- und -fördereinrichtung und zur Verbindung der Kammer 18 mit der Absaugeinrichtung können jeweils Schlauchleitungen vorgesehen sein, die über Anschlußstutzen anzuschließen sind. Als Luftklimatisierungs- und -fördereinrichtung und als Absaugeinrichtung sind handelsübliche Baugruppen nutzbar, so daß eine nähere Erläuterung hier entfallen kann.

Mit der Ausgestaltung nach Fig.4 wird ebenfalls wie schon mit der Anordnung nach Fig.2 erreicht, daß die Ablationsprodukte abgesaugt werden, ohne daß diese den Laserstrahl 3 durchqueren und so die Intensität der Laserstrahlung beeinträchtigen können. Die Oberfläche des Gegenstandes 2 kann aufgrund des klimatisierten Luftstromes 7 nicht austrocknen, wodurch gleichbleibende Abtragungsbedingungen gewährleistet sind.

## Patentansprüche

1. Anordnung zum Abtragen von Material mit Hilfe von Laserstrahlung von der Oberfläche eines Gegenstandes (2), nämlich durch photorefraktive Keratektomie, wobei
- mittels einer Luftfördereinrichtung und einer Absaugeinrichtung während der Dauer des Abtragungsprozesses ein Luftstrom über den Abtragungsort hinweg geführt wird,
**dadurch gekennzeichnet, daß**
- die Richtung der auf die Oberfläche zuströmenden Luft mit der über dem Abtragungsort an die Oberfläche gelegten Tangente einen Winkel von 0° bis 70° einschließt,
- die Strömungsgeschwindigkeit für den Luftstrom in einem Wertebereich von 1 m/s bis 10m/s vorgesehen ist,
- die Richtung der von der Oberfläche abströmenden Luft mit der Tangente über dem Abtragungsort einen Winkel von 0° bis 70° einschließt,
- Mittel zur Vorauswahl und eine Einrichtung zum Verändern der Strömungsgeschwindigkeit der über den Abtragungsort hinweg strömenden Luft aus einem vorgegebenen Wertebereich vor Beginn bzw. während des Abtragungsprozesses vorgesehen sind.

2. Anordnung nach Anspruch 1, wobei die Luftfördereinrichtung mit Austrittsöffnungen (6) und die Absaugeinrichtung mit Eintrittsöffnungen (10) ausgestattet sind und wobei die Luftförderung und die Vorgabe der Strömungsrichtung der Luft bewirkt wird durch zwei in Richtung des Laserstrahles (3) aufeinander folgende, ringförmig zentrisch um den Laserstrahl angeordnete Strömungskanäle (4, 8), von denen einer mit den Austrittsöffnungen (6), der andere mit den Eintrittsöffnungen (10) für den Luftstrom versehen ist.

3. Anordnung nach Anspruch 1 oder 2, **dadurch gekennzeichnet, daß** Mittel zur Vorauswahl der Temperatur und/oder der relativen Luftfeuchte aus vorgegebenen Wertebereichen vor Beginn des Abtragungsprozesses vorgesehen sind.

4. Anordnung nach einem der vorgenannten Ansprüche, **dadurch gekennzeichnet, daß** Einrichtungen zum Konstanthalten
- der Temperatur bei minus 8°C,
- der relativen Luftfeuchte bei 80%, und/oder
- der Strömungsgeschwindigkeit bei 3 m/s
der während des Abtragungsprozesses über den Abtragungsort hinweg strömenden Luft vorhanden sind.

5. Anordnung nach einem der Ansprüche 1 bis 3, **dadurch gekennzeichnet, daß** Einrichtungen zum Verändern der Temperatur und/oder der relativen Luftfeuchte während des Abtragungsprozesses innerhalb vorgegebener Wertebereiche vorgesehen sind.

6. Anordnung nach Anspruch 5, **dadurch gekennzeichnet, daß**
- eine Veränderung der Strömungsgeschwindigkeit innerhalb des Wertebereiches von 1 m/s bis 10 m/s vorgesehen ist,
- eine Lufterwärmungseinrichtung vorhanden und eine Veränderung der Temperatur innerhalb des Wertebereich von minus 20°C bis plus 30°C vorgesehen ist, und/oder
- eine Luftbefeuchtungseinrichtung vorhanden und eine Veränderung der relativen Luftfeuchte im Wertebereich von 0 bis 100% vorgesehen ist.

7. Anordnung nach Anspruch 6, **dadurch gekennzeichnet, daß** die Luftbefeuchtungseinrichtung mit einem Vernebler ausgestattet ist, der 0,5 ml bis 2 ml Wasser pro Minute vernebelt bei einer Tröpfchengröße von <4 µm.

8. Anordnung nach einem der Ansprüche 5 bis 7, **dadurch gekennzeichnet, daß** Ansteuerschaltungen vorhanden sind, durch welche Veränderungen von Temperatur, relativer Luftfeuchte und/oder Strömungsgeschwindigkeit während des Abtragungsprozesses anhand vorgegebener Zeitplan-Funktionen veranlaßt werden.

9. Anordnung nach einem der Ansprüche 5 bis 8, **dadurch gekennzeichnet, daß** Meßwertaufnehmer zur Messung von Temperatur- und/oder Feuchtigkeitswerten aus der unmittelbaren Umgebung des Abtragungsortes vorgesehen und über Auswerteeinrichtungen mit Ansteuerschaltungen verknüpft sind, durch die Veränderungen von Temperatur, relativer Luftfeuchte und/oder Strömungsgeschwindigkeit in Abhängigkeit von den gemessenen Werten veranlaßt werden.

10. Anordnung nach Anspruch 9, **dadurch gekennzeichnet, daß** zum Messen von Feuchtigkeitswerten eine Streulichtmeßeinrichtung vorgesehen ist, bei der die Intensität der Reflexion eines Laserstrahls mit Wellenlängen im sichtbaren oder infraroten Spektralbereich an der Materialoberfläche als Maß der Feuchtigkeit an der Materialoberfläche genutzt wird.

11. Anordnung nach Anspruch 9, **dadurch gekennzeichnet, daß** zum kontaktfreien Messen der aktuellen Temperaturen an der Materialoberfläche eine Thermokamera (14) vorgesehen ist.

## Claims

1. An arrangement for the ablation of material from the surface of an object (2) by means of laser radiation, that is to say by photorefractive keratectomy, with
- an air flow being guided across the ablation site for the duration of the ablation process by means of an air conveying device and a suction device,
**characterised in that**
- the direction of the air flowing towards the surface and the tangent to the surface at the ablation site include an angle of 0° to 70°,
- the flow velocity of the air flow is intended to be in a range from 1 m/s to 10m/s,
- the direction of the air flowing away from the surface and the tangent at the ablation site include an angle of 0° to 70°,
- means are provided for preselecting, within a specified range, the flow velocity of the air flowing over the ablation site before the start of the ablation process, and for varying the said flow velocity during the ablation process.

2. The arrangement as claimed in Claim 1, with the air conveying device being provided with outlet openings (6) and the suction device being provided with entrance openings (10), and with air conveyance and the definition of the flowing direction of the air being effected by two flow channels (4, 8) arranged consecutively in the direction of the laser beam (3) and shaped as rings centred around the laser beam, one of them being provided with the outlet openings (6) and the other one with the entrance openings (10) for the air flow.

3. The arrangement as claimed in Claim 1 or 2, **characterised in that** means are provided for preselecting the temperature and/or the relative air humidity within specified ranges before the start of the ablation process.

4. The arrangement as claimed in any of the previous claims, **characterised in that** means are provided for constantly keeping the air flowing across the ablation site during the ablation process
- at a temperature of minus 8°C,
- at a relative air humidity of 80%, and/or
- at a flow velocity of 3 m/s.

5. The arrangement as claimed in any of Claims 1 to 3, **characterised in that** means are provided for varying the temperature and/or the relative air humidity within specified ranges during the ablation process.

6. The arrangement as claimed in Claim 5, **characterised in that**
- means are provided for varying the flow velocity within a range of 1 m/s to 10 m/s,
- an air heating device is provided, and means are provided for varying the temperature within a range of minus 20°C to plus 30°C, and/or
- an air humidifying device is provided, and means are provided for varying the relative humidity within a range of 0 to 100%.

7. The arrangement as claimed in Claim 6, **characterised in that** the air humidifying device is provided with a nebuliser that nebulises 0.5 ml to 2 ml of water per minute with a droplet size of <4 µm.

8. The arrangement as claimed in any of Claims 5 to 7, **characterised in that** control circuits are provided that can, during the ablation process, effect changes of temperature, relative humidity and/or flow velocity according to specified time schedule functions.

9. The arrangement as claimed in any of Claims 5 to 8, **characterised in that** sensors for measuring temperature and/or humidity values from the direct environment of the ablation site are provided and, via evaluation devices, are coupled with control circuits by which changes of temperature, relative humidity and/or flow velocity are effected as functions of the measured values.

10. The arrangement as claimed in Claim 9, **characterised in that**, for measuring humidity values, a stray-light measuring device is provided in which the intensity of the reflection of a laser beam from the material surface is used as a measure of humidity, the said laser beam having wavelengths in the visible or infrared spectral range.

11. The arrangement as claimed in Claim 9, **characterised in that** a thermal camera (14) is provided for the contactless measurement of current temperatures on the material surface.

## Revendications

1. Dispositif permettant une ablation de matière au moyen d'un rayonnement laser sur la surface d'un objet (2), à savoir par kératectomie photoréfractive,
- un flux d'air se déplace au-dessus du site d'ablation pendant la durée du processus d'ablation au moyen d'un dispositif de transport de l'air et d'un dispositif d'aspiration,
**caractérisé en ce que**
- la direction de l'air affluant sur la surface forme un angle de 0° à 70° avec la tangente tracée au niveau de la surface au-dessus du site d'ablation,
- la vitesse de circulation du flux d'air est prévue dans une plage de valeurs de 1 m/s à 10 m/s,
- la direction du flux d'air s'échappant forme un angle de 0° à 70° avec la tangente au-dessus du site d'ablation,
- il est prévu des moyens de présélection et un dispositif permettant de modifier, dans une plage de valeurs prédéfinie, la vitesse de circulation de l'air se déplaçant au-dessus du site d'ablation, avant le début ou pendant le processus d'ablation.

2. Dispositif selon la revendication 1, dans lequel le dispositif de transport de l'air est muni d'ouvertures de sortie (6) et le dispositif d'aspiration est muni d'ouvertures d'entrée (10), et dans lequel le transport de l'air et la définition du sens de circulation de l'air sont induits par deux canaux de circulation (4, 8), qui se suivent dans la direction du faisceau laser (3), sont disposés en anneau de manière centrée autour du faisceau laser et parmi lesquels l'un est muni des ouvertures de sortie (6) et l'autre est muni des ouvertures d'entrée (10) pour le flux d'air.

3. Dispositif selon la revendication 1 ou 2, **caractérisé en ce qu'**il est prévu des moyens pour la présélection de la température et/ou de l'humidité relative de l'air dans des plages de valeurs prédéfinies, avant le début du processus d'ablation.

4. Dispositif selon l'une quelconque des revendications précédentes, **caractérisé en ce que** sont présents des dispositifs pour maintenir de manière constante
- la température à moins 8°C,
- l'humidité relative de l'air à 80 %, et/ou
- la vitesse de circulation à 3 m/s,
de l'air se déplaçant au-dessus du site d'ablation pendant le processus d'ablation.

5. Dispositif selon l'une quelconque des revendications 1 à 3, **caractérisé en ce qu'**il est prévu des dispositifs pour modifier la température et/ou l'humidité relative de l'air à l'intérieur des plages de valeurs prédéfinies, pendant le processus d'ablation.

6. Dispositif selon la revendication 5, **caractérisé en ce que**
- une modification de la vitesse de circulation à l'intérieur de la plage de valeurs de 1 m/s à 10 m/s est prévue,
- un dispositif de chauffage de l'air est présent et une modification de la température à l'intérieur de la plage de valeurs de moins 20°C à plus 30°C est prévue, et/ou
- un dispositif d'humidification de l'air est présent et une modification de l'humidité relative de l'air dans la plage de valeurs de 0 à 100 % est prévue.

7. Dispositif selon la revendication 6, **caractérisé en ce que** le dispositif d'humidification de l'air est équipé d'un nébuliseur qui nébulise 0,5 ml à 2 ml d'eau par minute avec une grosseur de gouttes inférieure à 4 µm.

8. Dispositif selon l'une quelconque des revendications 5 à 7, **caractérisé en ce que** des circuits de commande sont présents, par lesquels les modifications de la température, de l'humidité relative de l'air et/ou de la vitesse de circulation sont ordonnées pendant le processus d'ablation à l'appui de fonctions de grille horaire.

9. Dispositif selon l'une quelconque des revendications 5 à 8, **caractérisé en ce que** des éléments de mesure destinés à mesurer la température et/ou l'humidité relative de l'air dans l'environnement immédiat du site d'ablation sont prévus et sont reliés via des dispositifs d'analyse à des circuits de commande, par lesquels les modifications de la température, de l'humidité relative de l'air et/ou de la vitesse de circulation sont ordonnées en fonction des valeurs mesurées.

10. Dispositif selon la revendication 9, **caractérisé en ce que** pour mesurer les valeurs d'humidité, il est prévu un dispositif de mesure de la lumière diffusée, dans lequel l'intensité de la réflexion d'un faisceau laser avec des longueurs d'ondes dans le domaine du spectre visible et infrarouge à la surface de la matière est utilisée en tant que mesure de l'humidité à la surface de la matière.

11. Dispositif selon la revendication 9, **caractérisé en ce qu'**il est prévu une caméra thermique (14) pour la mesure sans contact des températures actuelles à la surface de la matière.
